# EUROPÄISCHE PATENTSCHRIFT

(11) **EP 0 629 439 B1**
(45) Veröffentlichungstag und Bekanntmachung des Hinweises auf die Patenterteilung: **29.01.1997**
(21) Anmeldenummer: 94108930.2
(22) Anmeldetag: 10.06.1994
(51) Int. Cl.: B01J 23/42, B01J 27/045, B01J 37/03, B01J 37/20, C01B 21/14

(54) **Platin auf Aktivkohle enthaltender sulfidierter Katalysator**
Sulphuretted catalyst of platinum on activated carbon
Catalyseur sulfuré de platine sur du charbone actif

(30) Priorität: 14.06.1993 DE 4319648; 26.04.1994 DE 4414491
(43) Veröffentlichungstag der Anmeldung: 21.12.1994
(73) Patentinhaber: Degussa Aktiengesellschaft, 60311 Frankfurt (DE)
(72) Erfinder: Bartels, Karin, Dr., D-63456 Hanau (DE); Deller, Klaus, Dr., D-63512 Hainburg (DE); Despeyroux, Bertrand, Dr., F-78112 Fourqueux (FR); Simon, Jochen, D-63579 Freigericht (FR)

(56) Entgegenhaltungen:
- EP-A- 0 002 651
- EP-A- 0 467 174
- EP-A- 0 467 192
- FR-A- 2 303 598
- US-A- 3 138 560
- PATENT ABSTRACTS OF JAPAN vol. 12, no. 265 (E-637) 23. Juli 1988 & JP-A-63 048 761 (FUJI ELECTRIC CO LTD) 1. März 1988

## Beschreibung

Die Erfindung betrifft einen Platin auf Aktivkohle enthaltenden sulfidierten Katalysator und ein Verfahren zu seiner Herstellung.

Sulfidierte edelmetallhaltige Katalysatoren werden seit Jahrzehnten großtechnisch hergestellt und angewandt. In der DE-PS 21 50 220 wird zum Beispiel ein Verfahren zur Herstellung eines solchen Katalysators beschrieben. Durch Sulfidierung des edelmetallhaltigen Katalysators mit einer schwefelhaltigen chemischen Verbindung wird durch partielle Vergiftung des Katalysators eine höhere Selektivität in der gewünschten chemischen Reaktion bei in der Regel verminderter Katalysatoraktivität erzielt. Über den Mechanismus der Sulfidierung selbst, die Natur des gebundenen Schwefels an der Katalysatoroberfläche sowie die Interaktion zwischen Schwefel, Edelmetall und Träger ist wenig bekannt.

Besonders bei der selektiven Hydrierung von Halogennitroaromaten (siehe zum Beispiel die nicht vorveröffentlichte deutsche Patentanmeldung P 42 18 866.0 auch veröffentlicht als EP-A-573910) und der reduktiven Alkylierung (DE-OS 27 36 228) werden Platin als Edelmetall und Aktivkohle als Träger enthaltende Katalysatoren vor anderen Katalysatorsystemen bevorzugt.

Die Selektivität dieser Katalysatoren in diesen Reaktionen wird durch teilweise Vergiftung durch Sulfidierung verbessert.

Als Sulfidierungsmittel werden Schwefel selbst oder weitere schwefelhaltige Verbindungen, wie H₂S, Dimethylsulfoxid, (NH₄)₂S, Na₂S, Na₂SO₄, wobei z. B. durch Einwirken einer starken Säure, wie H₂SO₄ der Schwefel der schwefelhaltigen Verbindung freigesetzt wird, verwendet. Hierbei werden Konzentrationen von 0,1 - 10 Mol Schwefel pro Mol Platin, vorzugsweise aber 0,1 - 2 Mol Schwefel pro Mol Platin angewendet.

Aufgabe der vorliegenden Erfindung ist es, einen gattungsgemäßen Katalysator sowie ein Verfahren zu seiner Herstellung anzugeben, der nach partieller Vergiftung durch Sulfidierung mit konventionellen Sulfidierungsmitteln eine deutlich verbesserte Katalysatorleistung als herkömmliche Katalysatoren aufweist.

Die Aufgabe wird durch einen Platin auf Aktivkohle enthaltenden sulfidierten Katalysator gelöst, der erhältlich ist durch Mischen einer wäßrigen Lösung einer Platinverbindung mit einer wäßrigen Suspension von Aktivkohle, Erwärmen der Mischung auf eine Temperatur zwischen 70 und 100° C, Ausfällen der Platinverbindung mit einer Base und Reduzieren der schwer löslichen Platinverbindung mit einem Reduktionsmittel unter Beibehaltung der Temperatur, Abfiltrieren und Waschen des so erhaltenen Katalysators, Behandeln des Katalysators mit einem Sulfidierungsmittel und erneutes Waschen des fertigen Katalysators, dadurch gekennzeichnet, daß die wäßrige Lösung der Platinverbindung ein Oxidationsmittel enthält.

Der Platingehalt des fertigen Katalysators kann zwischen 0,1 und 5 Gew.-% relativ zur Aktivkohle betragen. Der Zusatz eines Oxidationsmittels zu der wäßrigen Lösung der Platinverbindung vor dem Imprägnieren des pulvrigen Trägers aus Aktivkohle führt überraschenderweise zu einer platinhaltigen Phase, die die Katalysatorleistungen nach Sulfidierung mit konventionellen Sulfidierungsmitteln deutlich verbessert.

Erstaunlich dabei ist die Tatsache, daß der durch Oxidationsmittelzugabe hergestellte Katalysator vor Sulfidierung nicht unbedingt eine verbesserte Leistung in den katalytischen Reaktionen zeigt.

Als geeignete Aktivkohlenträger können säureaktivierte Kohlen und dampfaktivierte Kohlen mit oder ohne anschließender Säurewäsche eingesetzt werden. Zur Säureaktivierung werden üblicherweise Mineralsäuren wie Phosphorsäure oder Salpetersäure verwendet.

In Abhängigkeit von der Aktivierungsmethode und des eingesetzten Rohstoffes resultieren Aktivkohlen mit einem Restaschegehalt von 1 bis 5 Gew.-%, einem pH-Wert größer als 4 und mittleren Teilchendurchmessern zwischen 10 und 40 µm. Die spezifische Oberfläche der Aktivkohlen ist sehr groß und liegt gewöhnlich zwischen 500 und 1500 m²/g. Ihr Gesamtporenvolumen ist in der Regel größer als 0,5 ml/g.

Zur Bestimmung des pH-Wertes von Aktivkohle wird 1 g Aktivkohle in 100 ml Wasser suspendiert. Nach 5 Minuten Rühren wird der pH-Wert bei 25° C gemessen. Bevorzugt werden für den erfindungsgemäßen Katalysator Aktivkohlen mit einem pH-Wert größer als 5 eingesetzt.

Als geeignete Oxidationsmittel können anorganische Perverbindungen, wie Persulfate und Perchlorate, Natriumhypochlorit und Alkylhydroperoxide eingesetzt werden, insbesondere aber H₂O₂ in Konzentrationen von 0,1 - 10,0 Mol/Mol Platin, vorzugsweise aber 0,5 - 5,0 Mol/Mol Platin.

Als geeignete Sulfidierungsmittel können alle aus dem Stand der Technik bekannten schwefelhaltigen Verbindungen eingesetzt werden.

Beim Mischen der wäßrigen Lösung der Platinverbindung mit der wäßrigen Suspension von Aktivkohle kann es schon zu einer teilweisen Ausfällung einer schwer löslichen Platinverbindung kommen, die sich auf der Aktivkohle niederschlägt. Zum vollständigen Ausfällen des Platins als schwer lösliche Verbindung wird dem Gemisch aus platinhaltiger Lösung und Aktivkohle-Suspension eine Base zugegeben. Geeignete Basen sind Natriumkarbonat, Natriumhydroxid oder Kaliumhydroxid.

Eine weitere Aufgabe der Erfindung ist die Angabe eines Verfahrens zur Herstellung des sulfidierten Katalysators. Diese Aufgabe wird durch ein Verfahren gelöst, wonach man eine wäßrige Lösung einer Platinverbindung mit einer wäßrigen Suspension von Aktivkohle mischt, diese Mischung auf eine Temperatur zwischen 70 und 100° C erwärmt, die Platinverbindung mit einer Base ausfällt und die schwer lösliche Platinverbindung unter Beibehaltung der Temperatur mit einem Reduktionsmittel reduziert, den so erhaltenen Katalysator abfiltriert und wäscht, den Katalysator mit einem Sulfidierungsmittel behandelt und erneut wäscht. Das Verfahren ist dadurch gekennzeichnet, daß die wäßrige Lösung der Platinverbindung ein Oxidationsmittel enthält. Zum Waschen des Katalysators wird bevorzugt vollentsalztes Wasser (VE-Wasser) oder destilliertes Wasser eingesetzt.

Die wäßrige Suspension von Aktivkohle weist vorzugsweise eine Konzentration von 5 bis 30 Gew.-% auf. Es können verschiedene wasserlösliche Platinverbindungen eingesetzt werden, besonders bewährt hat sich Hexachloroplatinsäure-Hexahydrat.

Das Oxidationsmittel, bevorzugt Wasserstoffperoxid, wird in einer Menge von 0,1 - 10,0 Mol, vorzugsweise 0,5 - 5,0 Mol pro Mol Platin der Platinlösung zugegeben.

Aktivkohle-Suspension und Platinlösung werden miteinander vermischt und unter Rühren auf 70 bis 100° C erwärmt, bevor Platin durch Zugabe einer Base wie z. B. Natriumcarbonat oder Natronlauge in Form ihrer schwer löslichen Verbindungen auf der Aktivkohle niedergeschlagen und anschließend bei unveränderter Temperatur eventuell durch Zugabe eines Reduktionsmittels wie Hydrazin, Natriumformiat, Natriumboranat oder Formaldehyd, vorzugsweise Formaldehyd, reduziert wird.

Die Reihenfolge der Zugabe von Platinlösung und Base zur Aktivkohle-Suspension ist nicht zwingend. So kann die Base auch schon vor dem Einbringen der Platinlösung der Aktivkohle-Suspension zugemischt werden.

Nach Abfiltrieren und Waschen des so erhaltenen Pt/C-Katalysators wird das Sulfidierungsmittel wie z. B. Dimethylsulfoxid oder Ammoniumsulfid in Kombination mit einer starken Säure wie Schwefelsäure bei Normaltemperatur der wäßrigen Katalysator-Suspension zugesetzt.

Der sulfidierte Katalysator sollte einen Platingehalt von 0,1 - 5,0, vorzugsweise 0,5 - 3,5 Gew.-% und einen Schwefelgehalt von 0,1 - 10, vorzugsweise 0,1 - 2 Mol/Mol Platin aufweisen.

Als Alternative zur oben dargestellten Verfahrensweise, bei der die oxidationsmittel- und platinhaltige Lösung zu einer Aktivkohlen-Suspension zugegeben wird, kann die Aktivkohle zu der oxidationsmittel- und platinhaltigen Lösung zugegeben werden oder die oxidationsmittel- und platinhaltige Lösung auf die Aktivkohle versprüht werden.

Zur Stabilisierung der platinhaltigen Lösung kann es manchmal von Vorteil sein, der Lösung eine Mineralsäure, wie Salzsäure, Schwefelsäure oder Salpetersäure, zuzusetzen.

Die Erfindung wird nun anhand einiger Beispiele erläutert. Beispiel 1 beschreibt die Herstellung von erfindungsgemäßen Katalysatoren und Vergleichskatalysatoren aus dem Stand der Technik. Die unsulfidierte Vorstufe der Katalysatoren wurde in Beispiel 2 in einem Niederdrucktest auf Aktivität hin untersucht und miteinander verglichen. In Beispiel 3 wurden die erfindungsgemäßen sulfidierten Katalysatoren in einem Hochdrucktest auf Aktivität und Selektivität hin untersucht und mit den Vergleichskatalysatoren verglichen.

### Beispiel 1: Katalysator-Herstellung

Für alle Katalysatoren wurde als Träger Aktivkohle mit folgenden Materialdaten eingesetzt:

| Material: | pulvrige Aktivkohle |
|---|---|
| Spez. Oberfläche BET: | 1.500 m²/g (ASTM-D-3663) |
| Gesamtporenvolumen: | 1,5 ml/g (ASTM-D-4284) |
| Mittlere Korngröße: | 22 µm (ASTM-D-4464) |
| pH-Wert: | 10 |
| Aschegehalt: | < 2 % |

A) Herstellen der Katalysator-Vorstufen: (Pt+H₂O₂)/C (ohne Sulfidierung)
   Zur Herstellung eines Katalysators mit 3 % Platin-Beladung wurden 97 g Aktivkohle (Trockengewicht) in destilliertes Wasser bei einer Rührgeschwindigkeit von 300 U/min eingerührt. Dieser Suspension wurden 12 g einer 25 %igen wäßrigen Lösung von Hexachloroplatinsäure-Hexahydrat (entspricht 3 g Pt) mit 3 ml einer 30 %igen wäßrigen H₂O₂-Lösung zugegeben, entsprechend 2 Mol H₂O₂ pro Mol Platin.
   Anschließend wurde die Suspension auf 80° C erwärmt und zur Ausfällung schwer löslicher Hydroxide Natriumcarbonat unter ständigem Rühren zugefügt. Zur Reduktion des Niederschlags wurden schließlich 1,8 ml 37 %ige Formaldehydlösung zugegeben. Auch während der Reduktion wurde die Temperatur der Suspension auf 80° C konstant gehalten. Nach der Reduktion wurde der Katalysator über eine Nutsche abfiltriert und mit VE-Wasser gewaschen.
B) Herstellen des sulfidierten erfindungsgemäßen Katalysators: (Pt+H₂O₂+S)/C
   Zur Herstellung eines erfindungsgemäßen Katalysators mit 3 %iger Platinbeladung wurde zunächst ein Katalysator gemäß Teil A) präpariert und danach mit Dimethylsulfoxid gemäß dem Beispiel 1 aus der DE-PS 21 50 220 sulfidiert. Der Gesamtschwefelgehalt des sulfidierten Katalysators betrug etwa 0,4 Mol/S Mol Pt.
   Katalysatoren mit gleicher Platinbeladung, jedoch mit doppeltem bzw. vierfachem Schwefelgehalt, wurden durch entsprechende Vervielfachung des Sulfidierungsmittels hergestellt.
   Katalysatoren mit abweichenden Platinbeladungen wurden analog zum obigen Vorgehen durch gleichzeitige Vervielfachung der Mengen an Hexachloroplatinsäure, Natronlauge und Sulfidierungsmittel angefertigt.
C) Herstellen von Vergleichskatalysatoren: Pt/C
   Zum Vergleich mit dem erfindungsgemäßen Katalysator wurden platinhaltige Katalysatoren auf Aktivkohle, wie in Beispiel 1, Teil A) bzw. B) hergestellt, jedoch ohne Zugabe von H₂O₂.

### Beispiel 2: Niederdrucktestung der Katalysator-Vorstufen (ohne Sulfidierung)

Die Aktivität der Katalysator-Vorstufen wurde in der Hydrierung von Zimtsäure zu Dihydrozimtsäure unter folgenden Reaktionsbedingungen untersucht:

Zu 10 g Zimtsäure in 120 ml Ethanol wurden 200 mg Katalysator gegeben und in einem 250 ml Rührreaktor mit Begasungsrührer, Thermometer und Wasserstoffzufuhr überführt.

Der Reaktor wurde zunächst mit Stickstoff bei Normaldruck sorgfältig gespült. Dann wurde ein konstanter Wasserstoffdruck von 10 mbar über Normal bei einer Reaktionstemperatur von 25 ° C im Reaktor eingestellt, und der Wasserstoff über den Begasungsrührer (2000 U/min) gleichmäßig in der Lösung verteilt.

Zur Messung der Katalysatorleistung wurde die Katalysatoraktivität in der Hydrierreaktion von Zimtsäure zu Dihydrozimtsäure untersucht und in ml Wasserstoff/g Katalysator/min angegeben. Als Reaktionszeitraum für die Berechnung dieser Katalysatoraktivität diente die Reaktionszeit zwischen der 3. und 8. Minute nach Wasserstoffzufuhr.

Tabelle 1 gibt die Testergebnisse von Katalysatoren, die nach Beispiel 1, Teil A) bzw. Teil B) (ohne Sulfidierung) hergestellt wurden, wieder.

**Tabelle 1**

| Beispiel | Zusammensetzung | Katalysatoraktivität ml H₂/g Katalysator/min |
|---|---|---|
| 1 C | 3,0 % Pt/C | 245* (220 - 280) |
| 1 A | (3,0 % P + H₂O₂)C | 240* (230 - 270) |
| 1 C | 1,0 % Pt/C | 95* (80 - 120) |
| 1 A | 1,0 % Pt + H₂O₂)C | 90* (70 - 110) |

| | | |
|---|---|---|
| * Mittelwert nach Durchführung von 10 Versuchen | | |

Tabelle 1 zeigt, daß alle Katalysatorvorstufen eine vergleichbare Aktivität haben.

### Beispiel 3: Hochdrucktestung der Katalysatoren (nach Sulfidierung)

Aktivität und Selektivität der erfindungsgemäßen Katalysatoren nach Beispiel 1 B) wurden mit Aktivität und Selektivität von Vergleichskatalysatoren aus dem Stand der Technik gemäß Beispiel 1 C) bei der reduktiven Hydrierung von p-Aminodiphenylamin (PADPA) mit Methylisobutylketon (MIBK) verglichen.

Zu diesem Zweck wurden 3,77 Mol PADPA zusammen mit 4,63 Mol MIBK und 1,38 g eines 3 %igen Pt/C-Katalysators (entsprechend 0,2 Gew.-%, bezogen auf die Menge PADPA) in einen 2 l-Autoklaven gegeben. Der Autoklav wurde verschlossen, erst mit Stickstoff und dann mit Wasserstoff gespült und auf einen Wasserstoffdruck von 50 bar gebracht. Das Reaktionsgemisch wurde unter Rühren auf eine Temperatur von 190° C erwärmt. Nach 60, 90 und 120 Min. Reaktionszeit wurden Proben entnommen und analysiert.

Tabelle 2 enthält die Restgehalte der analysierten Proben an noch nicht umgesetztem PADPA sowie die benötigte Hydrierzeit, um den Restgehalt an PADPA unter 2 Gew.-% zu drücken.

**Tabelle 2**

| Beispiel | Hochdrucktest Rest PADPA-Gehalt (%) nach | | | Hydrierzeit [min.] bei Rest PADPA-Geh. < 2 % |
|---|---|---|---|---|
| | 60 min | 90 min | 120 min | |
| 1 C (3,0 Pt+S)/C | 7,5 | 3,8 | 2,0 | 120 |
| 1 C mit dopp. S-Geh. | 11,3 | 5,9 | 2,8 | 135 |
| 1 B (3,0 Pt+H₂O₂+S)C | 3,6 | 1,8 | 1,0 | 86 |
| 1 B mit dopp. S-Geh. | 4,2 | 2,0 | 1,1 | 90 |
| 1 B m.vierf.S-Geh. | 10,8 | 5,7 | 2,8 | 130 |

Tabelle 2 zeigt, daß durch Verwendung der erfindungsgemäßen Katalysatoren gemäß Beispiel 1 B) eine Aktivitätsverbesserung bis 35 % über dem Stand der Technik (Beispiel 1 C)) erreicht wurde. Die Selektivität wurde durch die H₂O₂-Behandlung nicht beeinfluß.

## Patentansprüche

1. Platin auf Aktivkohle enthaltender sulfidierter Katalysator mit einem Platingehalt von 0,1 bis 5,0 Gew.-% und einem Schwefelgehalt von 0,1 bis 2 Mol/Mol Platin, erhältlich durch Mischen einer wäßrigen Lösung einer Platinverbindung mit einer wäßrigen Suspension von Aktivkohle, Erwärmen der Mischung auf eine Temperatur zwischen 70 und 100° C, Ausfällen der Platinverbindung mit einer Base und Reduzieren der schwer löslichen Platinverbindung mit einem Reduktionsmittel unter Beibehaltung der Temperatur, Abfiltrieren und Waschen des so erhaltenen Produkts, Behandeln mit einem Sulfidierungsmittel und erneutes Waschen,
**dadurch gekennzeichnet**,
daß die wäßrige Lösung der Platinverbindung ein Oxidationsmittel enthält und daß die Aktivkohle einen mittleren Teilchendurchmesser von 10 bis 40 µm, eine spezifische Oberfläche von mehr als 500 m²/g, ein Gesamtporenvolumen von mehr als 0,5 ml/g, einen Restaschegehalt unter 5 Gew.-% und einen pH-Wert von mehr als 5 aufweist.

2. Verfahren zur Herstellung eines Platin auf Aktivkohle enthaltenden sulfidierten Katalysators nach Anspruch 1 durch Mischen einer wäßrigen Lösung einer Platinverbindung mit einer wäßrigen Suspension von Aktivkohle, Erwärmen dieser Mischung auf eine Temperatur zwischen 70 und 100° C, Ausfällen der Platinverbindung mit einer Base und Reduzieren der schwer löslichen Platinverbindung unter Beibehaltung der Temperatur mit einem Reduktionsmittel, Abfiltrieren und Waschen des so erhaltenen Produkts, Behandeln mit einem Sulfidierungsmittel und erneutes Waschen,
**dadurch gekennzeichnet**,
daß die wäßrige Lösung der Platinverbindung ein Oxidationsmittel enthält und daß die Aktivkohle einen mittleren Teilchendurchmesser von 10 bis 40 µm, eine spezifische Oberfläche von mehr als 500 m²/g, ein Gesamtporenvolumen von mehr als 0,5 ml/g, einen Restaschegehalt unter 5 Gew.-% und einen pH-Wert von mehr als 5 aufweist.

3. Verfahren nach Anspruch 2,
**dadurch gekennzeichnet**,
daß als Oxidationsmittel Wasserstoffperoxid in einer Menge von 0,1 bis 10,0 Mol, vorzugsweise 0,5 bis 5,0 Mol pro Mol Platin eingesetzt wird.

## Claims

1. A sulphidised catalyst which contains platinum on activated carbon, with a platinum content of 0.1 to 5.0 wt.% and a sulphur content of 0.1 to 2 moles per mole of platinum, obtainable by mixing an aqueous solution of a platinum compound with an aqueous suspension of activated carbon, heating the mixture to a temperature between 70 and 100°C, precipitating the platinum compound with a base and reducing the sparingly soluble platinum compound with a reducing agent at the same constant temperature, filtering off and washing the catalyst obtained in this way, treating with a sulphidising agent and washing again,
characterised in that the aqueous solution of platinum compound contains an oxidising agent and that the activated carbon has an average particle diameter of 10 to 40 µm, a specific surface area greater than 500 m²/g, a total pore volume greater than 0.5 ml/g, a residual ash content of less than 5 wt.% and a pH higher than 5.

2. A process for preparing a sulphidised catalyst which contains platinum on activated carbon according to Claim 1 by mixing an aqueous solution of a platinum compound with an aqueous suspension of activated carbon, heating this mixture to a temperature between 70 and 100°C, precipitating the platinum compound with a base and reducing the sparingly soluble platinum compound with a reducing agent at the same constant temperature, filtering off and washing the product obtained in this way, treating with a sulphidising agent and washing again, characterised in that the aqueous solution of platinum compound contains an oxidising agent and that the activated carbon has an average particle diameter of 10 to 40 µm, a specific surface area greater than 500 m²/g, a total pore volume greater than 0.5 ml/g, a residual ash content of less than 5 wt.% and a pH higher than 5.

3. A process according to Claim 2, characterised in that hydrogen peroxide in an amount of 0.1 to 10.0 moles, preferably 0.5 to 5.0 moles per mole of platinum is used as oxidising agent.

## Revendications

1. Catalyseur sulfuré contenant du platine sur charbon actif avec une teneur en platine de 0,1 à 5,0 % en poids et une teneur en soufre de 0,1 à 2 mol/mol de platine, qu'on peut obtenir en mélangeant une solution aqueuse d'un composé de platine et une suspension aqueuse de charbon actif, en chauffant le mélange à une température comprise entre 70 et 100°C, en précipitant le composé de platine avec une base et en réduisant le composé de platine peu soluble avec un agent réducteur en maintenant la température, en filtrant et lavant le produit ainsi obtenu, en traitant avec un agent de sulfuration et en lavant à nouveau,
caractérisé en ce que
la solution aqueuse du composé de platine contient un agent oxydant et que le charbon actif présente une taille de particule moyenne de 10 à 40 µm, une surface spécifique supérieure à 500 m²/g, un volume de pores global supérieur à 5.

2. Procédé de préparation d'un catalyseur sulfuré contenant du platine sur charbon actif selon la revendication 1, par mélange d'une solution aqueuse d'un composé du platine avec une suspension aqueuse de charbon actif, on chauffe ce mélange à une température comprise entre 70 et 100°C, on précipite le composé de platine avec une base et on réduit le composé de platine peu soluble avec un agent réducteur en maintenant la température, on filtre et lave le produit ainsi obtenu, on traite avec un agent de sulfuration et on lave à nouveau,
caractérisé en ce que
la solution aqueuse du composé de platine contient un agent oxydant et que le charbon actif présente une taille de particule moyenne de 10 à 40 µm, une surface spécifique supérieure à 500 m²/g, un volume de pores global supérieur à 0,5 ml/g, un taux de cendres inférieur à 5 % en poids et un pH supérieur à 5.

3. Procédé selon la revendication 2,
caractérisé en ce que
on utilise comme agent oxydant du peroxyde d'hydrogène en une quantité de 0,1 à 10,0 mol, de préférence 0,5 à 5,0 mol par mol de platine.
